# EUROPEAN PATENT APPLICATION

(11) **EP 2 740 935 A1**
(43) Date of publication of application: **11.06.2014**
(21) Application number: 12195714.6
(22) Date of filing: 05.12.2012
(51) Int. Cl.: F04B 43/06, A61B 5/04, A61B 5/0402, A61N 1/04, A61N 1/36, A61B 17/3203

(54) **Fluid device**

(71) Applicant: Smartbrain, 0201 Oslo (NO)
(72) Inventor: Sjaheim, Njål, Haldor, N-0352 Oslo (NO)
(74) Representative: Onsagers AS

(57) **Abstract**

The present invention comprises a device for providing a conductive fluid to a wet electrode, the device comprises at least one container (1) for storing a conductive fluid (2), said container comprises at least one controllable outlet (3), wherein the conductive fluid (2) is retainable in the container at an above ambient pressure, and the device is adapted to be integrated in a system for measuring a bio-potential on, and/or providing electric stimulation to, a skin surface of a subject.

## Description

### Technical field

The present invention concerns the field of wet electrodes, more specifically the provision of a system for delivery of electro-conductive fluids to a wet electrode system.

### Background of the invention

When choosing an electrode system in for instance electroencephalogram (EEG) measurements, there is a compromise between the time used for application, i.e. the time it takes to apply and prepare the electrodes onto the scalp of a subject, and the quality of the obtained measurements. The preferred electrodes are the so called wet electrodes, since these provide the best signal to noise ratio. The reason for the difficulties in obtaining a high quality signal is the low amplitude (µV) of the measured bio-potentials. By using wet electrodes, the electrode-skin impedance is lowered by using an electro-conductive fluid to obtain a better electrical contact between the electrode and skin. The contact is further depending on many different variables such as; the amount of dead skin cells (abrasion level), individual hair structure, length, volume, density, humidity and salinity level. In the physical recording there is also electrostatic discharge noise from electrode settling, electrochemical reactions between the electrode material, fluids and subjects biology. Due to these variables, and the required application of a conductive fluid, the application of wet electrodes is a highly time-consuming process compared to dry electrodes. It is estimated that a normal 64-channel EEG-montage requires one hour of skilled labour. The current "state of the art" connection-quality for both dry and wet electrodes is heavily dependent on the operator skills and technique. Even if two different montages seem similar at a glance, they can behave unpredictably because of differences in gel volume, surface area, dispersion and electrode-skin pressure. This problem can arise between individual electrodes in one subject, due to movement and uneven drying of the gel. This also poses a problem when the same subject is recorded in multiple sessions, making the recording of bio-potential more unpredictable than necessary.

The current state of the art commonly consists of applying the required electro-conductive fluid, for instance by using a syringe with blunted needle, followed by a manual adjustment of each individual electrode to lower the impedance (preferable below 10k ohm), between the skin and each electrode site. The manual adjustments are necessarily time-consuming because the process of hair and dead skin cell removal are in most subjects needed to obtain low impedances. Access to each electrode site, after applying the electrodes, is also required in order to accommodate the application of the conductive fluid. Simple access is not always possible.

Patent application US 2011/0288604 Al discloses a system for the automatic release of a conductive fluid in connection with a defibrillator. The fluid is contained in a receptacle and is being released by igniting a gas cartridge which causes a dose of fluid to burst.

The goal of the present invention is to alleviate or avoid at least some of the disadvantages of the present wet electrode techniques.

### Summary of the invention

The present invention provides a device comprising a container within which a conductive fluid is stored or contained. The device is suitable for incorporation into for instance an EEG-cap, or similar, used in an EEG/TES system, used in the recording of bio-potentials. The device can also be fitted with other fixing arrangements, as self-adhesive plasters for Electrocardiography (ECG), Electromyography (EMG) or other bio-potential applications. The fluid is kept at a pressure above ambient atmospheric pressure. Said pressure is preferably high enough to allow for a full discharge of the conductive fluid. The container comprises at least one outlet in fluid contact with the conductive fluid. When not in use, the outlet is in a closed state preventing the conductive fluid from exiting the container. The outlet may comprise any suitable means for regulating the flow of conductive fluid through a passage from the interior of the container to the exterior of the container. Such means may comprise any suitable type of valve, membrane, plug or similar, which is able to close the outlet until release of the fluid is desired. Said means are made up of, or are in combination with, any suitable actuator and/or transducer. When desired, the conductive fluid is released from the container at a suitable pressure, the pressure of the fluid is preferably sufficient to remove the *stratum corneum,* i.e. the outermost layer of the epidermis consisting of dead skin cells, in the same process. The dispersion pattern of the released fluid could be made in a way that provide a consistent penetration level and covered surface area regardless of hair density- and volume. The device may therefore provide a predictable and repeatable skin-electrode connection with equal dispersion and volume of gel in all recording sites.

The present invention provides the possibility of acquiring bio-potential's, faster and easier without compromising signal quality. This is especially important with multiple electrode Electroencephalogram (EEG) recordings and the application of Trans-cranial Electric Stimulation (TES) or High Definition focal-targeted Trans-cranial Electric Stimulation (HD-TES. This technique requires stable skin-electrode impedance and a predictable conductive surface area for electrical stimulation. The present innovation is not limited by the type of non-invasive electrodes and is adaptable to all bio-potential recording or stimulation where an easy and reliable skin-electrode connection is beneficiary. The future of digital EEG and TES is promising, but the standardization and elimination of variables in electrode montages is critical for cross-validation, usability and competitiveness.

The present invention is further defined in the following and the attached claims:
In one embodiment, the invention provide a device for providing conductive fluid to a wet electrode, the device comprises at least one container for storing a conductive fluid, said container comprises at least one controllable outlet, wherein the conductive fluid is retainable in the container at an above ambient pressure and the device is adapted to be integrated in a system for measuring a bio-potential on,
and/or providing electric stimulation to, a skin surface of a subject. Depending on the system for which the device is to be adapted, the container and/or outlet may be designed in any suitable shape or form, and may comprise any suitable connecting means, to make the device integrable in the system. The required shape, form or
connecting means, will vary depending on the specific system to be used, but will easily be inferred by a skilled person based on common knowledge and routine experiments. The device is intended to be integrated into the system during use.

The system for measuring a bio-potential is preferably suitable for EEG, ECG, EMG, TES and/or HD-TES.

In some embodiments of the invention, the interior of the container is divided in a first and a second section by a membrane, the first section containing a propellant fluid and the second section able to contain the conductive fluid, the conductive fluid being in fluid contact with at least one outlet.

In some embodiments of the invention, the interior of the container is divided in a first and a second section by a piston, the first section containing a propellant fluid and the second section able to contain the conductive fluid, the conductive fluid being in fluid contact with at least one outlet.

In some embodiments of the invention, the container, when in use, comprises an elastic membrane in contact with the conductive fluid, said conductive fluid pressurized by the compressive forces of said membrane. In these cases the device according to the invention does not require a propellant fluid to pressurize the conductive fluid. When the container does not store any conductive fluid, the membrane is not elastically extended. When the container is being filled with conductive fluid the elastic membrane will extend and exert a compressive force on the fluid. The fluid will in this manner be kept pressurized until the outlet is opened during use. The elastic membrane may constitute an integral part of the container, or divide an interior of the container in two sections. In the latter case, the container will comprise a first section with the conductive fluid in fluid contact with the outlet, and a second section. The second section may comprise a compressed gas if the second section is not in fluid contact with the surroundings. The gas of the second section may be pressurized before the conductive fluid is filled into the first section, or it may be pressurized due to the expansion of the first section.

In some embodiments of the invention, the propellant fluid is a compressed gas.

In some embodiments of the invention, the outlet described above is a remote controlled valve.

In a preferred embodiment of the device according to the invention, the pressure of the conductive fluid is sufficient to provide a flow, or jet, through the outlet, said flow, or jet, able to remove at least parts of the *stratum corneum.*

In an embodiment of the device according to the invention, the device comprises multiple containers. Each container will then contain a separate amount of conductive fluid in contact with a corresponding outlet.

In some embodiments according to the present invention, the device is adapted to be integrated into a membrane, an electrode cap or an electrode pad. Said membrane, electrode cap, or pad, is a part of a system for measuring a bio-potential on, and/or providing electric stimulation to, a skin surface of a subject.

Further, the present invention comprises the use of a device, as described above, in a system for measuring a bio-potential on, and/or providing electric stimulation to, a skin surface of a subject.

The present invention also provides an electrode for measuring a bio-potential on, and/or providing electric stimulation to, a skin surface of a subject, said electrode comprises a device for providing conductive fluid as described above.

Further, the present invention provides a system for measuring a bio-potential, wherein said system comprises an electrode, and a device for providing conductive fluid as described above.

In a final embodiment, the present invention provides a method for measuring a bio-potential on, and/or providing electric stimulation to, a skin surface of a subject, comprising the following step:
- applying a conductive fluid to the skin surface at a velocity or pressure sufficient to remove at least parts of the stratum corneum.

The method for measuring a bio-potential on, and/or providing electric stimulation to, a skin surface of a subject may also comprise at least one of the following steps:
- applying at least one electrode to the skin surface; and
- applying at least one device according to the invention close to the skin surface; and
- measuring the bio-potential on, or providing electric stimulation to, the skin surface.

The regulating means may comprise any suitable type of transducer, actuator or combination of both, that may release the conductive fluid instantly or controllably. The fluid regulating actuator, for instance in a valve, may work with any force and the interaction between them, for instance; electrical, mechanical, electromagnetic (including light), chemical, acoustic, thermal or similar in any combination with any type of external or internal energy source. A transducer could be used to convert targeted energy to power and activate the actuator for instance by converting differential pressure, wave energy, or similar. The regulating means or actuator could optionally be remote controlled, or activated for instance in response to a signal from an electrode, or a remote transmitter could send wireless signal to a transducer picking up targeted energy and thus working as a local actuator and energy source for the actuator. When used in the present description, the term "valve" is intended to comprise any type of assembly suitable for regulating the fluid flow through the outlet of the container. Such valves may preferably be a common electromechanical valve, but other types may also be advantageous in various settings or applications, such as a Piezoelectric type valve, a thermal release type valve wherein a thermo sensitive valve or decomposition of a material is actuated by a heating coil or similar, a chemical type valve where a reaction releases the pressure, a mechanical valve that is actuated by differential pressure or have an upper pressure limit actuated by additional pressure to the reservoir, or a resonant material that break up by an external signal having an appropriate wavelength.

### Short description of the drawings

Fig. 1 shows a schematic cross-section of an embodiment of the invention.
Fig. 2 shows a schematic cross-section of an embodiment of the invention.
Fig. 3 shows a schematic cross-section of an embodiment of the invention.
Fig. 4 shows a perspective view of the embodiment in fig. 3.
Fig. 5 shows a further embodiment of the invention.
Fig. 6 shows the embodiment in fig. 5.
Fig. 7 shows a schematic drawing of devices according to the invention incorporated into a membrane for qEEG recordings.

### Detailed description of the drawings

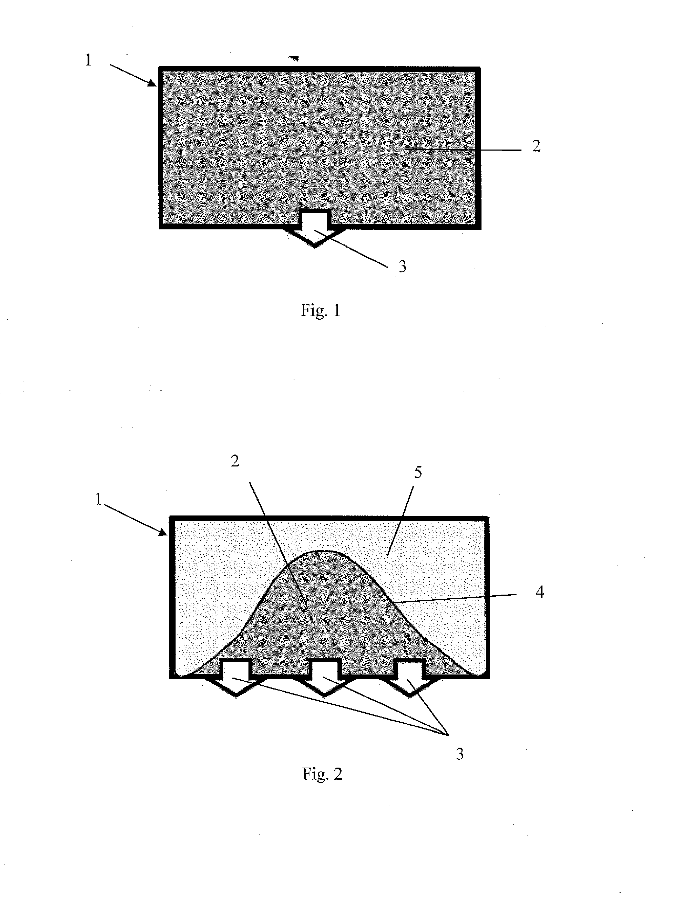

A schematic drawing of a device for providing conductive fluid 2 is shown in fig. 1. The device is suitable for incorporation into for instance a membrane, or skull cap, in a system for the recording of bio-potentials. The device comprises a fluid-proof container 1 enclosing the conductive fluid. The conductive fluid is kept at a pressure above ambient, i.e. above the atmospheric pressure. The container comprises an outlet 3. The outlet 3 is able to control or regulate the flow of conductive fluid 2 from the interior of the container to its exterior. The outlet may comprise any appropriate type of controllable valve, membrane or similar, often in connection with a nozzle. The flow through the outlet may be controlled by any suitable means, for instance by a connected electric signal or a remote radio signal. The pressure of the conductive fluid is preferably high enough to cause the fluid to be ejected through the outlet as a jet or flow sufficient to abrade the skin such that the layer of dead skin cells (the *stratum corneum*) is removed, optionally at least parts of said layer is removed. The pressure required of the conductive fluid, when stored in the container, to obtain such an abrasive effect is dependent on the type of outlet used. In the embodiment shown in fig. 1, the pressure of the conductive fluid is obtained by for instance formulating the fluid together with an appropriate propellant and a gelator.

In the device shown in fig. 2, the interior of the container 1 is divided into two compartments. One compartment contains a suitable propellant 5, such as a compressed gas, and the other compartment contains a conductive fluid 2. The two compartments are separated by a membrane 4. The membrane is fluid-proof and flexible. The propellant pressurizes the conductive fluid via the membrane 4. As shown in the specific embodiment of fig. 2, the membrane 4 need not be elastic. In this particular embodiment, the device has three separate outlets 3. The number of outlets may be varied according to need. The different outlets may be regulated separately or simultaneously, i.e. each outlet may be opened and/or closed independent of the other outlets, or not. In a further embodiment similar to the one shown in fig. 2, the membrane is elastic. In the case of an elastic membrane, the device does not require a propellant. The introduction of conductive fluid will in this case extend the membrane to accommodate the fluid volume and as a consequence the membrane will pressurize the fluid due to the compressive forces applied from the membrane on the fluid.

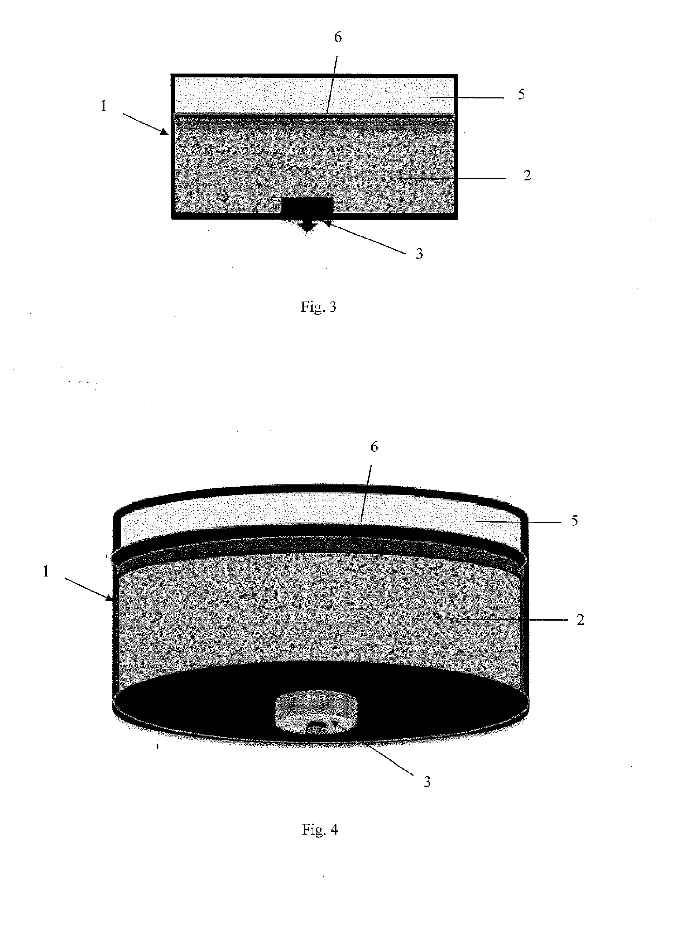

A further embodiment of a device according to the invention is shown in fig. 3. In this embodiment, the container 1 is divided into two compartments. One compartment contains a suitable propellant 5, such as a compressed gas, and the other compartment contains a conductive fluid 2. The two compartments are separated by a fluid-proof piston 6. The propellant pressurizes the conductive fluid via the piston 6. A perspective view of the device in fig. 3 is shown in fig. 4.

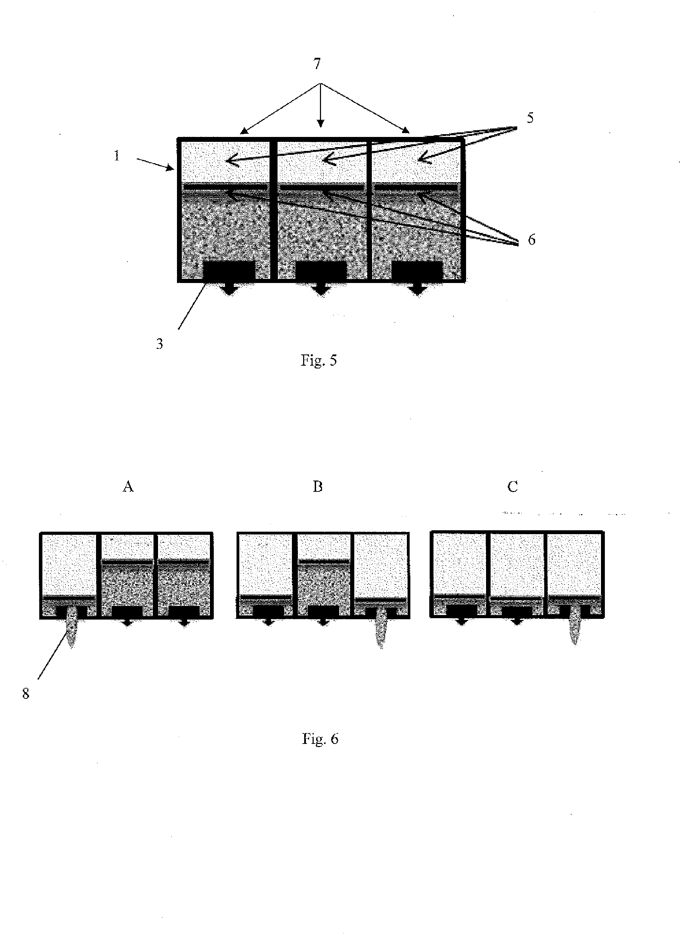

Fig. 5 shows a device comprising three containers, or described in another way; a device wherein the container 1 comprises three separate chambers 7, each chamber 7 comprising two compartments separated by a piston 6. Each pair of separated compartments consists of one compartment containing a propellant 5 and one compartment containing a conductive fluid. Each chamber has an outlet in fluid contact with the conductive fluid. The various outlets may be controlled independently of each other. In fig. 6, the device in fig. 5 is shown in three different stages A, B and C. A first outlet is opened in stage A, causing the formation of a jet 8 of conductive fluid. If the achieved bio-potential is too weak, stages B and C may be initiated to obtain lower skin-electrode impedance.

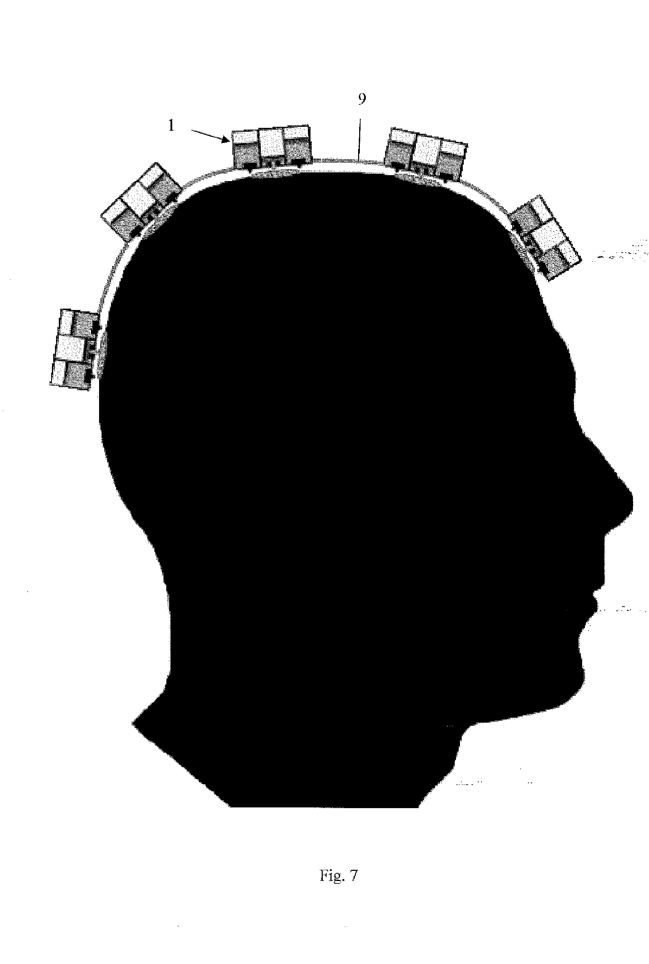

As described above, the device is primarily intended to be incorporated into a system for measuring a bio-potential, for instance a qEEG (quantitative EEG) recording system. A schematic drawing of such a system is shown in fig. 5. The device according to the invention is here incorporated into the membrane 9 of a cap arranged on the skull of a subject, is shown in fig. 7.

In use, the device may optionally be separate from the electrode measuring the bio-potential, or it may be an integral part of the electrode.

The device according to the invention may dispense conductive fluid in a single burst, in multiple bursts over time or any other advantageous schedule.

The fluid pressure is advantageously set to provide a flow-/jet-velocity able to abrade at least parts of the *stratum corneum.* Adequate flow-/jet-velocity of the fluid will contribute to a good bio-potential signal independent of possible obstacles such as dirt and hair.

The conductive fluid may be any fluid able to conduct electric signals compatible to the intended use. Such fluids comprise for instance a saline liquid or gel. To enhance the abrasive effect of the fluid, it may also comprise small particles.

When used separate from the electrode, the container of the device may be in any suitable material, such as a metal, a synthetic polymer etc.

When used as an integrated part of an electrode, the container of the device may advantageously be made in a conductive material, and being a part of, or constituting, the element of the electrode being in contact with the skin of a subj ect.

The container may be of any design which makes it suitable to be integrated into a system for measuring a bio-potential. In addition to the membrane or skull cap, described above with reference to fig. 7, other suitable systems for integration may be for instance electrode pads.

## Claims

1. A device for providing a conductive fluid to a wet electrode, the device comprises at least one container (1) for storing a conductive fluid (2), said container comprises at least one controllable outlet (3), wherein the conductive fluid (2) is retainable in the container at an above ambient pressure, and the device is adapted to be integrated in a system for measuring a bio-potential on, and/or providing electric stimulation to, a skin surface of a subject.

2. A device according to claim 1, wherein the device, during use, is integrated in a system for measuring a bio-potential on, and/or providing electric stimulation to, a skin surface of a subject.

3. A device according to claim 1 or 2, wherein the system is for recording EEG, ECG or EMG, or for TES or HD-TES.

4. A device according to claim 1 or 2, wherein the interior of the container is divided in a first and a second section by a membrane (4), the first section able to contain a propellant fluid (5), and the second section able to contain the conductive fluid (2) and being in fluid contact with the at least one outlet (3).

5. A device according to claim 1 or 2, wherein the interior of the container is divided in a first and a second section by a piston (6), the first section able to contain a propellant fluid (5), and the second section able to contain the conductive fluid (2) and being in fluid contact with the at least one outlet (3).

6. A device according to claim 1 or 2, wherein the container comprises an elastic membrane able to pressurize a retained conductive fluid, the fluid being in fluid contact with the at least one outlet (3), when the membrane is extended.

7. A device according to claim 4 or 5, wherein the propellant fluid (5) is a compressed gas.

8. A device according to any of the preceding claims, wherein the outlet (3) is a remote controlled valve.

9. A device according to any of the preceding claims, wherein the pressure of the conductive fluid (2) is sufficient to provide a flow (8), or jet, through the outlet (3), said flow, or jet, able to remove at least parts of the *stratum corneum* of a subject.

10. A device according to any of the preceding claims, comprising multiple containers (1).

11. A device according to any of the preceding claims, wherein said device is adapted to be integrated into a layer, such as a membrane, into an electrode cap or an electrode pad.

12. Use of a device according to any of the preceding claims in a system for measuring a bio-potential on, and/or providing electric stimulation to, a skin surface of a subject.

13. An electrode for measuring a bio-potential on, and/or providing electric stimulation to, a skin surface of a subject, said electrode comprising a device according to any of claims 1-10.

14. A system for measuring a bio-potential on, and/or providing electric stimulation to, a skin surface of a subject, said system comprising an electrode, and a device according to any of claims 1-10.

15. A method of measuring a bio-potential on, and/or providing electric stimulation to, a skin surface of a subject, comprising the step of:
- applying a conductive fluid to the skin surface at a velocity or pressure sufficient to remove at least parts of the stratum corneum.
